# FASCICULE DE BREVET EUROPEEN

(11) **EP 1 097 131 B1**
(45) Date de publication et mention de la délivrance du brevet: **05.10.2005**
(21) Numéro de dépôt: 99929421.8
(22) Date de dépôt: 07.07.1999
(51) Int. Cl.: C07C 323/52, C07C 319/28

(54) **PROCEDE DE SEPARATION DE L'ACIDE HYDROXYMETHYLTHIOBUTYRIQUE**
VERFAHREN ZUR ABTRENNUNG VON HYDROXYMETHYLTHIOBUTTERSÄURE
METHOD FOR SEPARATING HYDROXYMETHYLTHIOBUTYRIC ACID

(30) Priorité: 10.07.1998 FR 9808874
(43) Date de publication de la demande: 09.05.2001
(73) Titulaire: Adisseo Ireland Limited, Dublin 1 (IE)
(72) Inventeur: CARENCOTTE, Frédéric, F-69330 Meyzieu (FR); GARRAIT, Michel, F-69390 Millery (FR); GROS, Georges, F-92160 Antony (FR)
(74) Mandataire: Guerre, Dominique
(86) Numéro de dépôt international: PCT/FR1999/001636
(87) Numéro de publication internationale: WO 2000/002853

(56) Documents cités:
- EP-A- 0 143 100
- EP-A- 0 330 527
- WO-A-97/23452
- DE-A- 2 727 409
- US-A- 3 175 000

## Description

La présente invention concerne un procédé amélioré de préparation de l'acide 2-hydroxy-4-méthylthiobutyrique (HMTBA) sous forme de solution aqueuse, et plus spécifiquement un procédé de séparation améliorée à partir d'un mélange d'hydrolysat acide du 2-hydroxy-4-méthylthiobutyronitrile (HMTBN). L'acide 2-hydroxy-4-méthylthiobutyrique, analogue de la I-méthionine, est utilisé en nutrition animale.

Cummins décrit dans le brevet US 3,773,927 un procédé dans lequel le HMTBA est produit par hydrolyse du HMTBN par l'acide chlorhydrique dans des conditions telles que la suspension produite contienne du chlorure d'ammonium solide qui est éliminé par centrifugation. Le filtrat est ensuite distillé sous vide pour éliminer l'eau. Ce brevet US 3,773,927 décrit un procédé de préparation d'un produit liquide aqueux à forte concentration en acide HMTBA (85 à 90% en masse). Ce type de produit obtenu par un tel procédé possède une forte odeur et une couleur sombre, et contient des oligomères d'ester. Ces caractéristiques sont probablement dues aux conditions de haute température appliquées à un produit à faible teneur en eau lors de la dernière étape de déshydratation. Les autres inconvénients de ce procédé sont une forte consommation énergétique lors de cette même étape et des difficultés et pertes de rendement lors de la centrifugation ou de la filtration.

Le brevet US 2,745,745 décrit la séparation du HMTBA par extraction avec une phase organique non miscible à l'eau comme le diéthyl éther. Le brevet anglais No. 915,193 décrit un procédé de préparation de sels de calcium du HMTBA où ce-dernier est extrait par un éther, tel que l'isopropyl éther ou le butyl éther, ayant un point d'ébullition supérieur à celui de l'éthyl éther. De l'eau est ajoutée pour former une émulsion, à laquelle on ajoute du carbonate de calcium ou de l'hydroxyde de calcium pour précipiter le sel de calcium du HMTBA. Ce brevet ne concerne pas la préparation de produits liquides contenant essentiellement le HMTBA.

Gielkens mentionne dans le brevet US 3,175,000 que l'extraction directe du HMTBA de l'hydrolysat donne de mauvais rendements. Dans ce brevet, l'extraction n'est utilisée que comme moyen de récupération secondaire.

Le brevet US 4,524,077 décrit l'extraction liquide-liquide du HMTBA par un solvant organique non-miscible à l'eau, de préférence la méthylisobutyl cétone, puis la récupération du HMTBA à partir de l'extrait obtenu contenant au minimum 5% d'eau en poids, sur la base de la masse de HMTBA récupérée. Le produit ainsi préparé a une couleur plus claire, moins d'odeur, une plus faible viscosité et une meilleure stabilité thermique que les produits préparés par les procédés classiques décrits précédemment.

Le brevet EP 0 330 527 décrit un procédé de séparation de l'HMTBA consistant à former deux phases par addition à la solution d'hydrolyse d'ammoniaque, chacune des phases, la première organique contenant l'HMTBA et la seconde aqueuse contenant le sel d'ammonium sont ensuite soumises à une opération d'évaporation de façon à éliminer l'eau. La phase organique concentrée, filtrée, est ensuite diluée de façon à adapter sa concentration à celle de la solution commerciale. Le sulfate d'ammonium obtenu à partir de la phase aqueuse est pollué par des résidus soufrés, il présente souvent une mauvaise odeur qui rend sa commercialisation difficile ou qui demande des traitements accessoires pour le désodoriser. Le présent procédé exige une quantité d'énergie importante lors de l'évaporation de l'eau de chacune des phases obtenues après neutralisation et une étape de traitement accessoire du sulfate d'ammonium également couteuse. La présente invention a permis de résoudre les problèmes ci-dessus évoqués.

### Description de l'invention

La présente invention concerne un nouveau procédé de préparation de l'acide 2-hydroxy-4-méthylthiobutyrique (HMTBA), et plus spécifiquement un nouveau procédé de séparation du HMTBA. Ce procédé consiste dans une première étape à ajouter un agent de neutralisation, constitué d'hydroxyde d'ammonium, à la solution d'hydrolyse sulfurique du 2-hydroxy-4-méthylthiobutyronitrile ce qui entraîne, après décantation, la séparation du milieu en deux phases. Ces deux phases que l'on sépare et qui contiennent chacune de l'HMTBA et des sels, sont constituées d'une phase organique qui est optionnellement soumise à une étape de relargage des sels par addition d'un solvant organique et d'une phase aqueuse qui est épuisée en acide 2-hydroxy-4-méthylthiobutyrique résiduel par ajout d'un solvant organique.

Le système de relargage des sels de la phase organique est constitué par l'addition d'un solvant peu miscible à l'eau. Parmi ces solvants on peut citer tous les solvants permettant de solubiliser l'HMTBA, compatibles chimiquement avec le milieu et présentant peu d'affinité avec l'eau. Ils peuvent être choisis parmi les cétones, les aldéhydes, les éthers, les esters, les carbonates ou les alccols. Préférentiellement, il s'agit de cétones de bas poids moléculaire telle que la méthyléthylcétone, la méthylisobutylcétone (MIBK) ou d'éthers comme le méthyl tertiobutyl éther ou le diisopropyl éther ou de carbonates comme le diéthylcarbonate. Plus préférentiellement, il s'agit de la méthyl éthyl cétone, de la méthylisobutyl cétone et du carbonate d'éthyle.

Le système d'épuisement de l'HMTBA à partir de la phase aqueuse est constitué par l'addition d'un solvant peu miscible à l'eau. Parmi ces solvants on peut citer tous les solvants permettant de solubiliser l'HMTBA, compatibles chimiquement avec le milieu et présentant peu d'affinité avec l'eau. Ils peuvent être choisis parmi les cétones, les aldehydes, les éthers, les esters, les carbonates ou les alccols. Préférentiellement, il s'agit de cétones de bas poids moléculaire telle que la méthyléthylcétone, la méthylisobutylcétone (MIBK) ou d'éthers comme le méthyl tertiobutyl éther ou le diisopropyl éther ou de carbonates comme le diéthylcarbonate. Plus préférentiellement, il s'agit de la méthyl éthyl cétone, de la méthylisobutyl cétone et du carbonate d'éthyle.

Chacune des étapes relargage des sels de la phase organique et épuisement de la phase aqueuse peuvent être réalisées conjointement lors de la mise en oeuvre du procédé d'isolement de l'HMTBA. On préfère mettre ces deux étapes en oeuvre conjointement et dans un procédé continu.

Dans le cas où l'on veut relarguer les sels de la solution organique d'HMTBA, la quantité de solvant organique qui est ajoutée au milieu organique constitué d'HMTBA est selon un rapport pondéral solvant/solution organique de HMTBA de préférence supérieur à 0,3 et encore plus préférentiellement compris entre 0,3 et 1. Il est évident que l'homme de l'art adaptera la quantité de solvant à utiliser à l'économie du procédé. La température à laquelle est opérée le relargage est compatible avec la nature du solvant utilisé et notamment située en dessous de son point d'ébullition.

Pour épuiser l'HMTBA de la solution aqueuse, il est préférable, selon une meilleure manière de mettre en oeuvre l'invention, d'utiliser une quantité de solvant organique par rapport à la solution aqueuse contenant les sels inorganiques, notamment le sulfate d'ammonium, établi selon une quantité pondérale supérieure à 0,05 et de préférence comprise entre 0,1 et 0,5. La température à laquelle est opérée l'épuisement est compatible avec la nature du solvant utilisé et notamment située en dessous de son point d'ébullition.

L'étape suivante consiste à évaporer le ou les solvant(s) organique(s) utilisé(s) qui peuvent être similaires dans les deux étapes ou différents. Cette opération demande moins d'énergie que l'opération d'élimination de l'eau opérée dans l'art antérieur.

Après évaporation du ou des solvants qui sont éventuellement recyclés pour une nouvelle opération, on obtient une phase organique contenant peu d'eau et contenant une quantité très réduite de sels ce qui élimine la phase de filtration pratiquée auparavant. La mise au titre commercial de 88% en poids est ensuite réalisée par addition de l'eau nécessaire.

Après séparation biphasique entre le solvant organique d'épuisement et la phase aqueuse contenant les sels, on obtient une solution de sels et majoritairement de sulfate d'ammonium qui est cristallisée et ne contient pratiquement plus de polluants organiques. Les cristaux sont de meilleure qualité et ne présentent pratiquement plus d'odeurs.

Selon une variante du procédé, il comprend les étapes supplémentaires suivantes. La solution aqueuse de sels est traitée par électrodialyse afin de régénérer d'une part de l'ammoniac qui est recyclé à l'étape de neutralisation et d'autre part de l'acide sulfurique qui après concentration est recyclé à l'étape d'hydrolyse de l'HMTBN.

Selon une autre variante du procédé, il comprend les étapes supplémentaires suivantes. La solution aqueuse de sels, essentiellement constituées de sulfate d'ammonium est traitée par voie thermique dans une unité de régénération sulfurique de manière à récupérer de l'acide sulfurique concentré recyclable directemrent à l'hydrolyse de l'HMTBN.

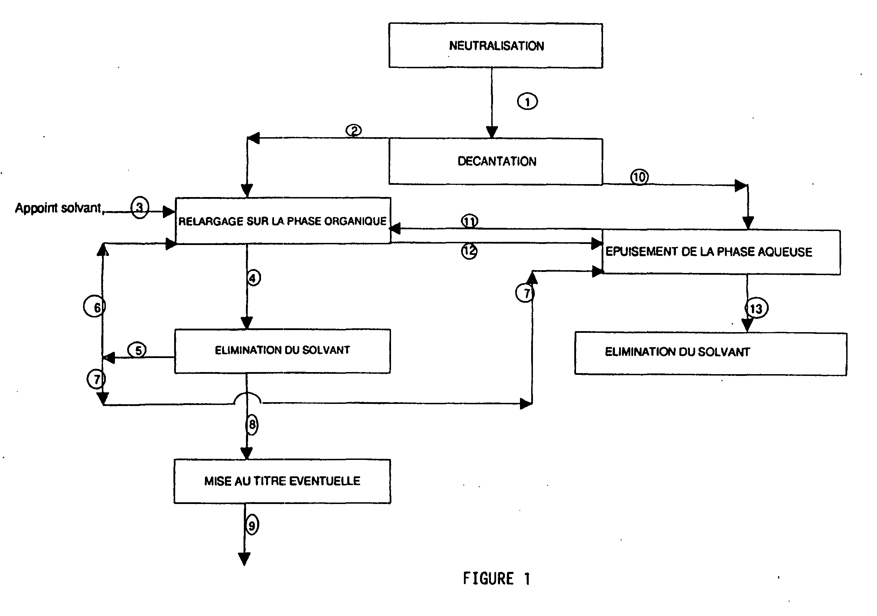

Une mise en oeuvre industrielle du procédé combiné est réalisée de la façon suivante (cf. figure 1)

Le flux neutralisé (flux 1) est décanté.

La phase organique issue de la décantation subit une opération de relargage des sels par un traitement avec une phase essentiellement composée de solvant organique (flux 6). Ce traitement consiste en un contact liquide-liquide. Il est réalisé dans un mélangeur décanteur classique ou dans tout autre contacteur liquide-liquide choisi parmi les appareils suivants : une batterie de mélangeurs-décanteurs, une colonne garnie, une colonne à plateaux perforés, une colonne à disques rotatifs, un extracteur centrifuge, une colonne pulsée ou tout autre contacteur liquide-liquide.

L'extrait et le raffinat sont séparés (flux 4 et flux 12). Le raffinat est recyclé à l'étape d'épuisement en HMTBA de la phase aqueuse issue de la décantation. L'extrait (flux 4) subit un traitement visant à éliminer le solvant. L'élimination est réalisée notamment par évaporation, distillation ou entraînement à la vapeur. Cette opération d'élimination est conduite pour obtenir un HMTBA en pied (flux 8) très appauvri en solvant résiduel et suffisamment concentré avant une mise au titre éventuelle. Le flux de tête (flux 5) provenant de cette élimination de solvant est recyclé
- en partie à l'étape de relargage sur la phase organique issue de la décantation(flux 6)
- en partie à l'étape d'épuisement sur la phase aqueuse issue de la décantation (flux 7)

La phase aqueuse décantée (flux 10) subit un traitement d'épuisement de l'HMTBA par contact avec une phase essentiellement composée de solvant organique (flux 7). Ce traitement est également réalisé dans un contacteur liquide-liquide, qui peut être une batterie de mélangeurs-décanteurs, une colonne garnie, une colonne à plateaux perforés, une colonne à disques rotatifs, un extracteur centrifuge, une colonne pulsée ou tout autre contacteur liquide - liquide.

L'extrait et le raffinat sont séparés (flux 11 et flux 13). L'extrait (flux 11) est recyclé à l'étape de relargage sur la phase organique issue de la décantation alors que le raffinat (flux 13) subit un traitement visant à éliminer le solvant. Ce traitement est notamment une distillation, une évaporation ou un stripping. Il est de préférence réalisé lors d'une cristallisation du sulfate d'ammonium par évaporation.

La présente invention sera plus complètement décrite à l'aide des exemples suivants qui ne doivent pas être considérés comme limitatifs de l'invention.

### 1- Exemples d'extraction liquide-liquide réalisée sur la phase aqueuse après décantation du flux d'hydrolyse neutralisé

L'objectif de cette opération est d'extraire l'HMTBA résiduel présent dans la phase aqueuse après décantation avant cristallisation du sulfate d'ammonium.
La composition de la phase aqueuse à extraire est la suivante :
[HMTBA] = 3.7%P/P
[EAU] = 40.9%P/P
[sels] = 55.4%P/P

### Exemple d'épuisement de cette phase aqueuse par la MIBK :

505.9 grammes de ce flux de phase aqueuse ont été mis en contact dans un réacteur agité de 1 litre avec 56.2 grammes de MIBK sous une température de 75°C. Le ratio solvant d'extraction/solution à séparer était de 11.1%P/P. Après décantation des deux phases à 75°C, les deux phases ont été prélevées et analysées :
La phase supérieure dans laquelle l'HMTBA est en partie extrait représentait 67.9 grammes et était chargée à 20.5%P/P en HMTBA. Dans cette phase supérieure, la teneur en sels était inférieure à la limite de détection de l'outil analytique (potentiométrie)
Ainsi, 74.3% de l'HMTBA engagés ont pu être extrait par de la MIBK en un seul étage avec un rapport solvant organique engagé/solution à extraire de 11.1%
La phase inférieure était quant à elle chargée à 0.97%P/P en HMTBA.
Le coefficient de partage exprimé comme suit [HMTBA]phase supérieure/[HMTBA]phase inférieure=21.1.

### 2 - Exemple d'épuisement de la phase aqueuse par le carbonate d'éthyle :

505.9 grammes de phase aqueuse ont été mis en contact dans un réacteur agité de 1 litre avec 56.2 grammes de carbonate d'éthyle sous une température de 75°C. Le ratio solvant d'extraction/solution à séparer était de 11.1 %P/P. Après décantation des deux phases à 75°C, les deux phases ont été prélevées et analysées :
La phase supérieure dans laquelle l'HMTBA est en partie extrait représentait 67.3 grammes et était chargée à 14.2%P/P en HMTBA. Dans cette phase supérieure, la teneur en sels était inférieure à la limite de détection de l'outil analytique (potentiométrie)
Ainsi, 50.3% de l'HMTBA engagés ont pu être extraits par du carbonate d'éthyle en un seul étage avec un rapport solvant organique engagé/solution à extraire de 11.1 %
La phase inférieure était quant à elle chargée à 1.53%P/P en HMTBA.
Le coefficient de partage exprimé comme suit [HMTBA]phase supérieure/[HMTBA]phase inférieure=9:3.

### 3 - Exemple d'épuisement de la phase aqueuse par la Méthyl Ethyl Cétone

505.9 grammes de phase aqueuse ont été mis en contact dans un réacteur agité de 1 litre avec 56.2 grammes de MEC sous une température de 75°C. Le ratio solvant d'extraction/solution à séparer était de 11.1%P/P. Après décantation des deux phases à 75°C, les deux phases ont été prélevées et analysées :
La phase supérieure dans laquelle l'HMTBA est en partie extrait représentait 65.3 grammes et était chargée à 23.7%P/P en HMTBA. Dans cette phase supérieure, la teneur en sels était inférieure à la limite de détection de l'outil analytique (potentiométrie)
Ainsi, 82.9% de l'HMTBA engagés ont pu être extraits par de la MEC en un seul étage avec un rapport solvant organique engagé/solution à extraire de 11.1%
La phase inférieure était quant à elle chargée à 0.6%P/P en HMTBA.
Le coefficient de partage exprimé comme suit [HMTBA]phase supérieure/[HMTBA]phase inférieure=39.5.

### 4 - Exemples de relargage liquide-liquide réalisé sur la phase organique après décantation du flux d'hydrolyse neutralisé

L'objectif de cette opération est d'épurer la phase organique en sels ((NH4)2SO4 et NH4HS04) par déplacement de ces sels dans une phase aqueuse qui se forme par ajout d'un solvant présentant peu d'affinités avec l'eau.
La composition de la phase organique issue de la décantation du flux d'hydrolyse neutralisé est la suivante :
[HMTBA] = 66.6%P/P
[EAU] = 22.4%P/P
[sels] = 11%P/P

### Exemple d'extraction des sels par ajout de MIBK

379 grammes de ce flux de phase organique ont été mis en contact dans un réacteur agité de 1 litre avec 137.3 grammes de MIBK sous une température de 75°C. Le ratio solvant d'extraction/solution à séparer était de 36.2%P/P. Après décantation à 75°C, les deux phases formées ont été prélevées et analysées :
La phase supérieure dans laquelle l'HMTBA a été extrait représentait 445.7g et était chargée à 1% en sels. Ainsi, avec le rapport solvant organique engagé/solution à traiter utilisé, 89% des sels présents dans le milieu avant extraction ont pu être déplacés vers la phase aqueuse créée par l'ajout de MIBK.
La phase inférieure dans laquelle les sels sont en partie déplacés représentait 70.2 grammes et était chargée à 2%P/P en HMTBA. Ainsi, dans ces conditions de déplacement, 99.4% de l'HMTBA engagés ont pu être extraits par de la MIBK en un étage avec un rapport solvant organique engagé/solution à traiter de 36.2%.
La phase inférieure était quant à elle chargée à 53%P/P en sels.
Le coefficient de partage exprimé comme suit [sels]phase inférieure/[sels]phase supérieure=53.

### 5- Exemple d'extraction des sels par ajout de carbonate d'éthyle

379 grammes du flux de phase organique ont été mis en contact dans un réacteur agité de 1 litre avec 137.3 grammes de carbonate d'éthyle sous une température de 75°C. Le ratio solvant d'extraction/solution à séparer était de 36.2%P/P. Après décantation à 75°C, ies deux phases formées ont été prélevées et analysées :
La phase supérieure dans laquelle l'HMTBA a été extrait représentait 455.5g et était chargée à 2.4%P/P en sels. Ainsi, avec le rapport solvant organique engagé/solution à traiter utilisée, 73.9% des sels présents dans le milieu avant extraction ont pu être déplacés vers la phase aqueuse créée par l'ajout de carbonate d'éthyle.
La phase inférieure dans laquelle les sels sont en partie déplacés représentait 60.8 grammes et était chargée à 2.5%P/P en HMTBA. Ainsi, dans ces conditions de déplacement, 99.4% de l'HMTBA engagés ont pu être extraits par du carbonate d'éthyle en un étage avec un rapport solvant organique engagé/solution à traiter de 36.2%P/P.
La phase inférieure était quant à elle chargée à 50.6%P/P en sels.
Le coefficient de partage exprimé comme suit [sels]phase inférieure/[sels]phase supérieure=21.1.

### 6 ― Exemple d'extraction des sels par ajout de MEC

379 grammes du flux de phase organique ont été mis en contact dans un réacteur agité de 1 litre avec 137.3 grammes de MEC sous une température de 75°C. Le ratio solvant d'extraction/solution à séparer était de 36.2%P/P. Après décantation à 75°C, les deux phases formées ont été prélevées et analysées :
La phase supérieure dans laquelle l'HMTBA a été extrait représentait 467g et était chargée à 2.1%P/P en sels. Ainsi, avec le rapport solvant organique engagé/solution à traiter considéré, 76.5% des sels présents dans le milieu avant extraction ont pu être déplacés vers la phase aqueuse créée par l'ajout de MEC.
La phase inférieure dans laquelle les sels sont en partie déplacés représentait 49.3 grammes et était chargée à 2%P/P en HMTBA. Ainsi, dans ces conditions de déplacement, 99.5% de l'HMTBA engagés ont pu être extraits par du carbonate d'éthyle en un étage avec un rapport solvant organique engagé/solution à traiter de 36.2%P/P.
La phase inférieure était quant à elle chargée à 64.8%P/P en sels.
Le coefficient de partage exprimé comme suit [sels]phase inférieure/[sels]phase supérieùre=30.9.

## Revendications

1. Procédé pour séparer l'acide 2-hydroxy-4-méthylthiobutyrique (HMTBA) d'une solution d'hydrolyse sulfurique du 2-hydroxy-4-methylthiobutyronitrile (HMTBN), **caracterise en ce qu'**il comprend les étapes suivantes :
on neutralise la solution d'hydrolyse par addition d'hydroxyde d'ammonium,
on décante pour obtenir une phase organique et une phase aqueuse,
on sépare la phase organique et la phase aqueuse contenant chacune de l'HMTBA et des sels, et
on épuise l'HMTBA de la phase aqueuse par addition d'un solvant organique peu miscible à l'eau, en une quantité suffisante pour épuiser la quasi-totalité de l'HMTBA présent.

2. Procédé selon la revendication 1, **caracterisé en ce que**, après séparation des phases organique et aqueuse, on relargue les sels contenus dans la phase organique avec un solvant organique peu miscible à l'eau, en une quantité suffisante pour extraire la quasi-totalite de l'HMTBA présent.

3. Procédé selon la revendication 1, **caractérisé en ce que** le solvant pour épuiser l'HMTBA de la phase aqueuse et le solvant pour extraire l'HMTBA de la phase organique sont identiques ou différents.

4. Procédé selon la revendication 3, **caractérisé en ce que** le solvant pour épuiser l'HMTBA de la phase aqueuse et/ou le solvant pour extraire l'HMTHA de la phase organique sont choisis parmi les cétones, les aldéhydes, les éthers, les esters, les carbonates et les alcools.

5. Procédé selon la revendication 4, **caracterisé en ce que** le solvant est une cétone choisie parmi la méthyléthylcetone et la méthylisobutylcétone.

6. Procédé selon la revendication 4, **caracterisé en ce que** le solvant est un éther choisi parmi le méthyltertiobutyl éther et le diisopropyl éther.

7. Procédé selon la revendication 4, **caractérisé en ce que** le solvant est choisi parmi la méthyléthylcétone, la méthylisobutylcétone et le carbonate d'éthyle.

8. Procédé selon l'une quelconque des revendications précédentes, **caractérisé en ce que** la quantité de solvant suffisante pour épuiser l'HMTBA de la phase aqueuse est telle que le rapport en poids du solvant à la solution aqueuse obtenue après décantation est supérieure à 0,05.

9. Procédé selon la revendication 8, **caractérisé en ce que** la quantité de solvant est telle que ledit rapport est compris entre 0,1 et 0,5.

10. Procédé selon l'une quelconque des revendication 2 à 9, **caractérisé en ce que** la quantité de solvant suffisante pour extraire l'HMTBA de la phase organique est telle que le rapport en poids du solvant à la solution organique obtenue après décantation est supérieure à 0,3.

11. Procédé selon la revendication 10, **caractérisé en ce que** la quantité de solvant est telle que ledit rapport est compris entre 0,3 et 1.

12. Procédé selon l'une quelconque des revendications 2 à 11, **caractérisé en ce que** l'épuisement de HMTBA de la phase aqueuse et l'extraction de l'HMTBA de la phase organique sont effectués de façon concomitante et selon un procédé continu.

13. Procédé selon l'une quelconque des revendications 2 à 12, **caractérisé en ce qu'**après l'opération d'épuisement de HMTBA de la phase aqueuse et d'extraction de l'HMTBA de la phase organique, le solvant est évaporé et recyclé pour une nouvelle opération, la phase organique est mise au titre commercial par addition d'eau, la phase aqueuse épuisée est cristallisée.

14. Procédé selon la revendication 13, **caractérisé en ce qu'**il comprend les étapes supplémentaires consistant à traiter les cristaux récupérés à partir de la phase aqueuse, par électrodialyse pour régénérer d'une part de l'ammoniac et d'autre part de l'acide sulfurique, à recycler ledit ammoniac à l'étape de neutralisation et à recycler, après concentration, ledit acide sulfurique à l'étape d'hydrolyse de l'HMTBN.

15. Procédé selon la revendication 13, **caractérisé en ce qu'**il comprend les étapes supplémentaires consistant à traiter les cristaux récupérés à partir de la phase aqueuse, par voie thermique, dans une unité de régénération sulfurique pour récupérer une solution d'acide sulfurique, et à recycler ledit acide sulfurique à l'étape d'hydrolyse de l'HMTBN.

## Patentansprüche

1. Verfahren zur Abtrennung von 2-Hydroxy-4-methylthiobuttersäure (HMTBA) aus einer bei der Hydrolyse von 2-Hydroxy-4-methylthiobutyronitril (HMTBN) mit Schwefelsäure anfallenden Lösung, **dadurch gekennzeichnet, daß** man:
die Hydrolyselösung durch Zugabe von Ammoniumhydroxid neutralisiert,
absetzen läßt, wobei man eine organische Phase und eine wäßrige Phase erhält,
die organische Phase und die wäßrige Phase, die jeweils HMTBA und Salze enthalten, trennt und
das HMTBA durch Zugabe eines wenig wassermischbaren organischen Lösungsmittels in einer zur erschöpfenden Extraktion praktisch der gesamten vorliegenden HMTBA ausreichenden Menge erschöpfend aus der wäßrigen Phase extrahiert.

2. Verfahren nach Anspruch 1, **dadurch gekennzeichnet, daß** man nach der Trennung der organischen Phase und der wäßrigen Phase die in der organischen Phase enthaltenen Salze mit einem wenig wassermischbaren organischen Lösungsmittel in einer zur erschöpfenden Extraktion praktisch der gesamten vorliegenden HMTBA ausreichenden Menge freisetzt.

3. Verfahren nach Anspruch 1, **dadurch gekennzeichnet, daß** das zur erschöpfenden Extraktion von HMTBA aus der wäßrigen Phase verwendete Lösungsmittel und das zur erschöpfenden Extraktion von HMTBA aus der organischen Phase verwendete Lösungsmittel gleich oder verschieden sind.

4. Verfahren nach Anspruch 3, **dadurch gekennzeichnet, daß** das zur erschöpfenden Extraktion von HMTBA aus der wäßrigen Phase verwendete Lösungsmittel und/oder das zur erschöpfenden Extraktion von HMTBA aus der organischen Phase verwendete Lösungsmittel unter Ketonen, Aldehyden, Ethern, Estern, Carbonaten und Alkoholen ausgewählt werden.

5. Verfahren nach Anspruch 4, **dadurch gekennzeichnet, daß** es sich bei dem Lösungsmittel um ein unter Methylethylketon und Methylisobutylketon ausgewähltes Keton handelt.

6. Verfahren nach Anspruch 4, **dadurch gekennzeichnet, daß** es sich bei dem Lösungsmittel um einen unter Methyl-tert.-butylether und Diisopropylether ausgewählten Ether handelt.

7. Verfahren nach Anspruch 4, **dadurch gekennzeichnet, daß** das Lösungsmittel unter Methylethylketon, Methylisobutylketon und Diethylcarbonat ausgewählt wird.

8. Verfahren nach einem der vorhergehenden Ansprüche, **dadurch gekennzeichnet, daß** die zur erschöpfenden Extraktion der HMTBA aus der wäßrigen Phase ausreichende Lösungsmittelmenge so groß ist, daß das Gewichtsverhältnis des Lösungsmittels zur nach dem Absetzenlassen erhaltenen wäßrigen Lösung über 0,05 liegt.

9. Verfahren nach Anspruch 8, **dadurch gekennzeichnet, daß** die Lösungsmittelmenge so groß ist, daß das Verhältnis zwischen 0,1 und 0,5 liegt.

10. Verfahren nach einem der Ansprüche 2 bis 9, **dadurch gekennzeichnet, daß** die zur erschöpfenden Extraktion der HMTBA aus der organischen Phase ausreichende Lösungsmittelmenge so groß ist, daß das Gewichtsverhältnis des Lösungsmittels zur nach dem Absetzenlassen erhaltenen organischen Lösung über 0,3 liegt.

11. Verfahren nach Anspruch 10, **dadurch gekennzeichnet, daß** die Lösungsmittelmenge so groß ist, daß das Verhältnis zwischen 0,3 und 1 liegt.

12. Verfahren nach einem der Ansprüche 2 bis 11, **dadurch gekennzeichnet, daß** die erschöpfende Extraktion der HMTBA aus der wäßrigen Phase und die Extraktion der HMTBA aus der organischen Phase gleichzeitig und nach einem kontinuierlichen Verfahren durchgeführt werden.

13. Verfahren nach einem der Ansprüche 2 bis 12, **dadurch gekennzeichnet, daß** man nach dem Arbeitsgang der erschöpfenden Extraktion der HMTBA aus der wäßrigen Phase und der Extraktion der HMTBA aus der organischen Phase das Lösungsmittel verdampft und für einen neuen Arbeitsgang zurückführt, die organische Phase durch Zugabe von Wasser auf die handelsübliche Konzentration einstellt und die erschöpfend extrahierte wäßrige Phase kristallisiert.

14. Verfahren nach Anspruch 13, **dadurch gekennzeichnet, daß** man zusätzlich aus den aus der wäßrigen Phase gewonnenen Kristallen durch Elektrodialysebehandlung Ammoniak zur Rückführung zur Neutralisation und Schwefelsäure zur Rückführung zur HMTBN-Hydrolyse nach Aufkonzentrierung regeneriert.

15. Verfahren nach Anspruch 13, **dadurch gekennzeichnet, daß** man zusätzlich aus den aus der wäßrigen Phase gewonnenen Kristallen auf thermischem Wege in einer Schwefelsäureregenerationseinheit eine Schwefelsäurelösung zur Rückführung zur HMTBN-Hydrolyse gewinnt.

## Claims

1. Process for separating 2-hydroxy-4-methylthiobutyric acid (HMTBA) from a solution resulting from the hydrolysis by sulphuric acid of 2-hydroxy-4-methylthiobutyronitrile (HMTBN), **characterized in that** it comprises the following stages:
the hydrolysis solution is neutralized by addition of ammonium hydroxide,
settling is carried out in order to obtain an organic phase and an aqueous phase,
the organic phase and the aqueous phase, each comprising HMTBA and salts, are separated, and
the HMTBA is exhaustively removed from the aqueous phase by addition of an organic solvent which is not very miscible with water in an amount sufficient to exhaustively remove virtually all the HMTBA present.

2. Process according to Claim 1, **characterized in that**, after separation of the organic and aqueous phases, the salts present in the organic phase are forced out with an organic solvent which is not very miscible with water in an amount sufficient to extract virtually all the HMTBA present.

3. Process according to Claim 1, **characterized in that** the solvent for exhaustively removing the HMTBA from the aqueous phase and the solvent for extracting the HMTBA from the organic phase are identical or different.

4. Process according to Claim 3, **characterized in that** the solvent for exhaustively removing the HMTBA from the aqueous phase and/or the solvent for extracting the HMBTA from the organic phase are chosen from ketones, aldehydes, ethers, esters, carbonates and alcohols.

5. Process according to Claim 4, **characterized in that** the solvent is a ketone chosen from methyl ethyl ketone and methyl isobutyl ketone.

6. Process according to Claim 4, **characterized in that** the solvent is an ether chosen from methyl tert-butyl ether and diisopropyl ether.

7. Process according to Claim 4, **characterized in that** the solvent is chosen from methyl ethyl ketone, methyl isobutyl ketone and ethyl carbonate.

8. Process according to any one of the preceding claims, **characterized in that** the amount of solvent sufficient for exhaustively removing the HMTBA from the aqueous phase is such that the ratio by weight of the solvent to the aqueous solution obtained after separation by settling is greater than 0.05.

9. Process according to Claim 8, **characterized in that** the amount of solvent is such that the said ratio is between 0.1 and 0.5.

10. Process according to any one of Claims 2 to 9, **characterized in that** the amount of solvent sufficient for extracting the HMTBA from the organic phase is such that the ratio by weight of the solvent to the organic solution obtained after separation by settling is greater than 0.3.

11. Process according to Claim 10, **characterized in that** the amount of solvent is such that the said ratio is between 0.3 and 1.

12. Process according to any one of Claims 2 to 11, **characterized in that** the exhaustive removal of the HMTBA from the aqueous phase and the extraction of the HMBTA from the organic phase are carried out concomitantly and according to a continuous process.

13. Process according to any one of Claims 2 to 12, **characterized in that**, after the operation for the exhaustive removal of the HMTBA from the aqueous phase and for the extraction of the HMTBA from the organic phase, the solvent is evaporated and recycled for a further operation, the organic phase is brought to the commercial assay by addition of water and the exhausted aqueous phase is crystallized.

14. Process according to Claim 13, **characterized in that** it comprises the additional stages which consist in treating the crystals recovered from the aqueous phase by electrodialysis in order to regenerate, on the one hand, ammonia and, on the other hand, sulphuric acid, in recycling said ammonia to the neutralization stage and in recycling, after concentrating, the said sulphuric acid to the stage for the hydrolysis of the HMTBN.

15. Process according to Claim 13, **characterized in that** it comprises the additional stages which consist in treating the crystals recovered from the aqueous phase by the thermal route in a plant for the regeneration of sulphuric acid in order to recover a sulphuric acid solution and in recycling the said sulphuric acid to the stage for the hydrolysis of the HMTBN.
